# EUROPEAN PATENT SPECIFICATION

(11) **EP 2 140 259 B1**
(45) Date of publication and mention of the grant of the patent: **21.05.2014**
(21) Application number: 08750477.5
(22) Date of filing: 24.04.2008
(51) Int. Cl.: G01N 33/00

(54) **Calibrator for gas analyser**
Kalibrator für einen Gasanalysator
Étalonneur pour un analyseur de gaz

(30) Priority: 25.04.2007 GB 0707995
(43) Date of publication of application: 06.01.2010
(73) Proprietor: Kittiwake Procal Limited, Storrington West Sussex RH20 4NQ (GB)
(72) Inventor: HUTCHINSON, Robin, Peterborough PE2 7HU (GB)
(74) Representative: Davies, Gregory Mark
(86) International application number: PCT/GB2008/001443
(87) International publication number: WO 2008/132447

(56) References cited:
- WO-A-2005/052571
- WO-A-2007/087703
- DE-A1- 19 852 472
- US-A1- 2002 139 167
- US-A1- 2006 165 561
- WILBRING P ET AL: "A Portable Calibration Gas Generator for H2O HC1, NH3 and Mercury" INTERNET CITATION, [Online] XP003016251 Retrieved from the Internet: URL:http://www.ecochem.biz/Library/CEM_200 1_2.pdf> [retrieved on 2007-01-01]

## Description

The present invention relates to a calibrator for testing and re-calibration of emission systems such as continuous emission systems and/or automated measuring systems.

Stack gas analysis apparatus is widely available through utilisation of a number of different techniques and is commonly used, a specific example being passing radiation through the gas and measuring the intensity of the radiation thereby obtaining data permitting calculation of the concentration of known constituents of the stack gases. Gas analysers give constant readings for only a short period of time and hence require periodic calibration to provide reliable analysis. This is generally carried out by prior art systems wherein a reservoir of a liquid is heated to provide a vapour, generally a water vapour, which passes through a heated conduit, subsequently mixed with a test gas and/or air and is passed into the stack. However, there are disadvantages with such an arrangement, in particular relating to heating the liquid to provide vapour and the percentage of the liquid is turned to vapour being unreliable. Heating is achieved by heating the fluid within the reservoir and pumping this vapour fluid mix through the conduit which itself is heated to substantially maintain the liquid in vaporised form. However, severe limitations are apparent with such an arrangement as it is difficult to control the percentage of vapour and additionally it takes a significant period of time for the apparatus to become stable such that the calibration may be carried out.

A calibration system having a supply unit that delivers a liquid and a carrier gas to an external evaporator and that controls the temperature of that evaporator, wherein the evaporator is to be connected to a gas analyzer to be calibrated, has been presented by Wilbring and Schmaeh (Int. Conf. Emission Monitoring 2001, Arnhem (NL)).

According to the present invention, there is apparatus for calibrating a gas analyser, as defined in claim 1.

The heater arrangement preferably includes an electrical heater. The heater arrangement preferably substantially surrounds the compartment. The gas preferably comprises a test gas. This ensures a high degree of mixing of the vapour and test gas to a desired temperature.

The liquid delivery system preferably comprises a displacement pump, and even more preferably comprises a positive displacement syringe. The displacement pump is preferably operated by a means to cause a stepwise delivery of liquid, and preferably comprises a stepper motor. Using such an arrangement ensures careful control of the volume of fluid to be vaporised is maintained. The liquid delivery system preferably further comprises a conduit arranged to couple to said connector. The conduit preferably comprises a capillary tube.

The test gas delivery system preferably comprises a second conduit arranged to supply test gas and/or air to said gas analyser. The test gas and/or air is preferably controlled by at least one mass flow controller. The conduit arranged to transport said liquid and the second conduit are preferably retained in a flexible tube.

A significant advantage of the present invention is that it is not a requirement to heat the fluid until reaching the connector. It is therefore not a requirement to provide heating means along the conduit which means the apparatus may be significantly simpler to manufacture and additionally may provide a more compact arrangement enabling simple portability. Additionally, there is little or no condensation of the fluid from when it is vaporised to passing into the monitoring systems. Calibration is more accurate, and additionally the apparatus can be used quickly from turning on the apparatus as a small volume of liquid only must be vaporised at any one time. The arrangement is therefore more accurate, more simple, and enables a higher percentage of vapour to be provided by the calibration system.

Both said first and second conduits are preferably retained in a flexible tube. The tube is preferably of a length of approximately two metres. This is suitable for the user of the apparatus to allow either access to the sample inlet point.

The apparatus is preferably maintained within a portable package wherein the flexible tube extends therefrom. Again, this provides the advantage that an operator can access areas where such monitoring systems are located with relative ease.

Embodiments of the present invention will now be described by way of example only with reference to the accompanying drawings in which:
Figure 1 is a schematic side view of a calibration apparatus according to an exemplary embodiment of the present invention.
Figure 2 is a schematic side view of a connector according to an exemplary embodiment of the present invention.

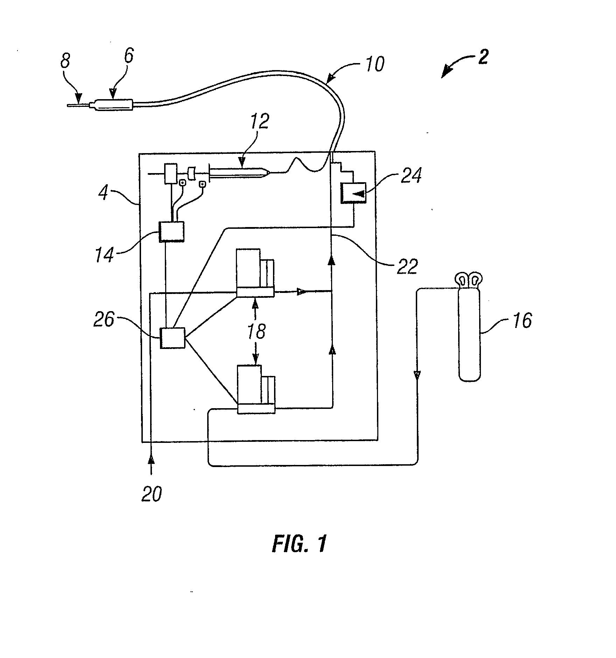

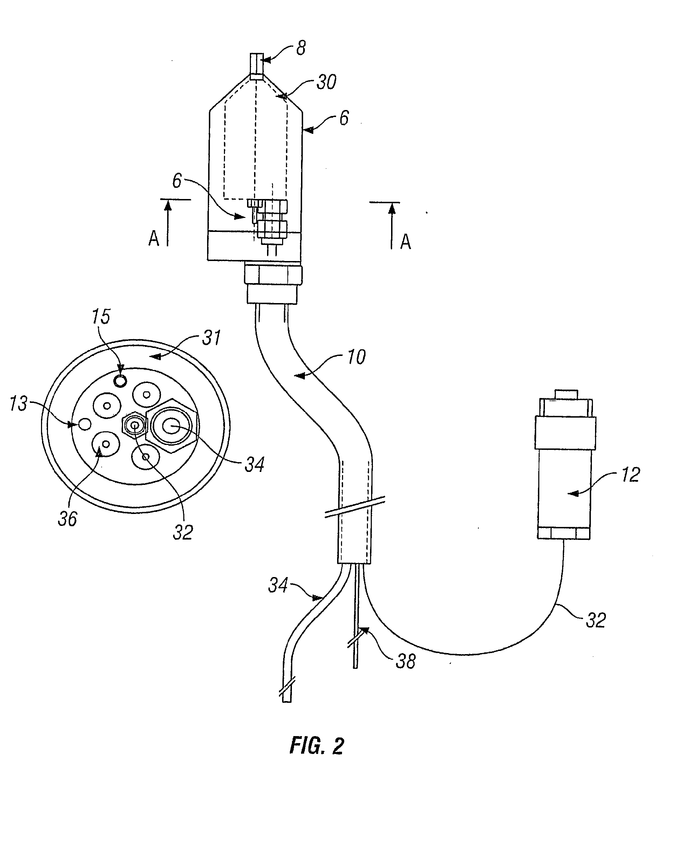

Referring to Figure 1, the apparatus 2 comprises a casing constructed from any light weight robust material such as polyurethane. Such an apparatus can then be carried by an operator on their back. The apparatus 2 comprises a connector 6 including a nozzle 8. The connector 6 is described in more detail with respect to Figure 2. However, the connector includes a heating arrangement selectable to ensure the desired percentage of water vapour is produced. A flexible tube 10 extends between the connector and the casing 4 and contains therein a capillary tube (not shown) and a conduit (not shown) for carrying liquid and gas and/or air as required. Water is injected into the capillary tube via displacement pump 12 which is controlled in a preferred embodiment by a stepper motor 14. This ensures the exact quantity of water is injected into the capillary tube for subsequent heating in the connector 6. The test gas container 16 is connectable to a mass flow controller 18 which is a standard unit known in the art. The mass flow controller for the test gas 16 controls flow of test gas to the connector 6. Air may also be provided through line 20 which again is controlled by a mass flow controller 18. The output through mass flow controller 18 may be joined to provide a single line 22 passing to the connector 6. The evaporator control 24 is also provided to control the heating of the fluid in the connector 6. This, along with the stepper motor 14 and mass flow controller 18 are controlled through a control unit 26. This control unit 26 comprises a microprocessor with a switch panel and LCD display for ease of viewing by an operator. The heating arrangement is located within the connector to allow most effective heating and control of the percentage vapour and temperature of vapour used in the calibration.

Referring to Figure 2, a detailed view of the connector is shown together with a crosssection of the area indicated AA. The connector 6 comprises a nozzle 8, and a compartment 30 into which liquid and/or gas through a capillary tube 32 and gas line 34 respectively are supplied. The compartment 30 is a heated block which causes the fluid to evaporate and puts the gas in an excited state. The vapour and gas are therefore mixed in the compartment 30. As can be seen with respect to the cross-sectional view, a number of heating elements 36 ensure heating of the liquid leaving the capillary tube 32 and the gas leaving the gas line 34. An earth connection is provided and a temperature sensor 13 is present. The vapour/gas mix leaving the nozzle 8 is therefore heated and mixed to the desired temperature without being allowed to cool by passing through any further conduits. An electrical supply 38 is also indicated on Figure 2, which is utilised as the heat source. The displacement pump 12 is also indicated on Figure 2. Referring again to the cross-sectional view, an insulating material 31 surrounds the inlet to the compartment 30.

In use, the liquid water from the displacement pump 12 is delivered to the connector local to the point of injection into the monitoring system which may for example be a continuous emission system and/or automated measuring system. Once vaporised, the water is mixed with the test gas and dilution gas as required. The dilution gas will be air inserted through line 20. Again, such an arrangement avoids the need for the calibrator to have a heated line as heating of the liquid and gas (although beneficial but not essential) occurs immediately prior to conducting the gas analysis. Rapid response and minimal warm up time are therefore achieved.

The specification of such an apparatus is that water vapour samples of up to 60% by volume. An accuracy of 1% of water vapour level over the range 10% - 60% is achieved and a supply of 230/100V, 60/60Hz is utilised. Such a unit will weigh approximately 10 kilos and water concentration in mg/nm³ and percentage, set point, actual concentration, evaporating temperature, individual mass flow and water rates are displayed. The connection to the continuous emission system in the nozzle may be 6mm or ¼ inch insulated tube.

Three specific performance operations are envisaged and described by way of example only:

### Calibrator - W

Water Vapour Generator - This enables the generation of water vapour up to 60% for one hour at gas flow rate of 5litres/minute. The water vapour concentrations can be adjusted in between 0 and 60% water vapour by volume.

### Calibrator - WD

Water Vapour Generator/Test Gas Divider - This enables the humidification of test gas up to a water vapour content of 60% and 5 litres per minute gas flow rate. The test gas and water vapour concentrations can be adjusted independently. The device can be used to verify the correct operation of extractive CEM's (continuous emission system) sample preparation components. If the system requires the removal of water vapour by introducing for example humidified Sulphur Dioxide (SO₂) it can be determined if a proportion of the soluble gas is also removed. The humidified gas should if possible be injected at the stack take off to fully challenge the sample system.

### Calibrator - D

Test Gas Divider - This enables linearisation test to be carried out on the CEM (continuous emission system) utilising only one cylinder of test gas per species. The gas is blended with air to enable the linearisation test in accordance with the requirements of both European EN14181 and US EPA. Typically one cylinder of test gas per range is connected to the calibrator in turn, (zero and four concentrations of each as cylinder) is applied and the resultant CEM's reading recorded. The saving in the test gas cylinders make the calibrator a very cost effective approach to linearisation testing.

## Claims

1. Apparatus for calibrating a gas analyser by delivering a test gas and vapour thereto, the apparatus comprising a connector (6) arranged to deliver a test gas and vapour, the apparatus also including:
a) a heater arrangement (30) to effect evaporation of a liquid to provide the vapour;
b) a liquid delivery system (12, 14) arranged to supply liquid to the heater arrangement;
c) a test gas delivery system (18) arranged to deliver test gas to the connector; and
d) a controller (26) for controlling the heater arrangement (30), liquid delivery system (12, 14), and test gas delivery system (18);
**characterised in that** the connector comprises a mixing compartment (30) for receipt of liquid and test gas and a nozzle (8) arranged and configured to locate in a receiving aperture in the gas analyser;
and **in that** the connector (6) includes said heater arrangement, such that vapour/test gas mix leaving the nozzle (8) is heated in the connector and delivered to the gas analyser without passing through any further conduits.

2. Apparatus according to claim 1, wherein said heater arrangement (30) includes an electrical heater.

3. Apparatus according to any preceding claim, wherein said heater arrangement (30) substantially surrounds the compartment (30)

4. Apparatus according to any preceding claim, wherein said gas comprises a test gas.

5. Apparatus according to any preceding claim, wherein said liquid delivery system comprises a displacement pump (12).

6. Apparatus according to claim 5, wherein said displacement pump (12) comprises a positive displacement syringe.

7. Apparatus according to claims 7, wherein said displacement pump (12) is operated by a means to cause stepwise delivery of liquid, and preferably wherein said means to cause stepwise delivery of said liquid comprises a stepper motor.

8. Apparatus according to any preceding claim, wherein said liquid delivery system further comprises a conduit (32) arranged to couple to said connector (6).

9. Apparatus according to claim 8, wherein said conduit comprises a capillary tube (32).

10. Apparatus according to any preceding claim wherein said test gas delivery system comprises a second conduit (34) arranged to supply test gas and/or air to said gas analyser, and preferably wherein said test gas and/or air is controlled by at least one mass flow controller.

11. A system according to claim 8 and 10, wherein said conduit (34) arranged to transport said liquid and the second conduit are retained in a flexible tube.

## Patentansprüche

1. Vorrichtung zum Kalibrieren eines Gasanalysators durch Zufuhr eines Prüfgases und von Dampf zu letzterem, wobei die Vorrichtung einen Verbinder (6) umfasst, der dazu angeordnet ist, ein Prüfgas und Dampf zuzuführen, wobei die Vorrichtung außerdem Folgendes enthält:
a) eine Heizeranordnung (30) zum Bewirken von Verdampfung einer Flüssigkeit zur Bereitstellung des Dampfes;
b) ein Flüssigkeitszufuhrsystem (12, 14), das zur Zufuhr einer Flüssigkeit zu der Heizeranordnung angeordnet ist;
c) ein Prüfgaszufuhrsystem (18), das zur Zufuhr von Prüfgas zu dem Verbinder angeordnet ist; und
d) einen Controller (26) zum Steuern der Heizeranordnung (30), des Flüssigkeitszufuhrsystems (12, 14) und des Prüfgaszufuhrsystems (18);
**dadurch gekennzeichnet, dass** der Verbinder einen Mischraum (30) zur Aufnahme von Flüssigkeit und Prüfgas und eine Düse (8), die zur Positionierung in einer Aufnahmeöffnung im Gasanalysator konfiguriert ist, umfasst;
und dass der Verbinder (6) die Heizeranordnung enthält, so dass die Düse (8) verlassendes Dampf/Prüfgas-Gemisch im Verbinder erwärmt wird und dem Gasanalysator ohne Hindurchleiten durch irgendwelche weiteren Leitungen zugeführt wird.

2. Vorrichtung nach Anspruch 1, wobei die Heizeranordnung (30) einen elektrischen Heizer umfasst.

3. Vorrichtung nach einem vorhergehenden Anspruch, wobei die Heizeranordnung (30) den Raum (30) im Wesentlichen umgibt.

4. Vorrichtung nach einem vorhergehenden Anspruch, wobei das Gas ein Prüfgas umfasst.

5. Vorrichtung nach einem vorhergehenden Anspruch, wobei das Flüssigkeitszufuhrsystem eine Verdrängerpumpe (12) umfasst.

6. Vorrichtung nach Anspruch 5, wobei die Verdrängerpumpe (12) eine Spritze nach dem Verdrängerprinzip umfasst.

7. Vorrichtung nach Anspruch 7, wobei die Verdrängerpumpe (12) durch eine Einrichtung zum Bewirken einer stufenweisen Zufuhr von Flüssigkeit betrieben wird und wobei vorzugsweise die Einrichtung zum Bewirken einer stufenweisen Zufuhr der Flüssigkeit einen Schrittmotor umfasst.

8. Vorrichtung nach einem vorhergehenden Anspruch, wobei das Flüssigkeitszufuhrsystem ferner eine Leitung (32) umfasst, die zur Kopplung an den Verbinder (6) angeordnet ist.

9. Vorrichtung nach Anspruch 8, wobei die Leitung ein Kapillarröhrchen (32) umfasst.

10. Vorrichtung nach einem vorhergehenden Anspruch, wobei das Prüfgaszufuhrsystem eine zweite Leitung (34) umfasst, die dazu angeordnet ist, dem Gasanalysator Prüfgas und/oder Luft zuzuführen, und wobei vorzugsweise das Prüfgas und/oder die Luft durch mindestens einen Massenfluss-Controller gesteuert wird.

11. System nach Anspruch 8 und 10, wobei die Leitung (34), die dazu angeordnet ist, die Flüssigkeit zu befördern, und die zweite Leitung in einem Schlauch festgehalten werden.

## Revendications

1. Appareil pour étalonner un analyseur de gaz en distribuant un gaz d'essai et une vapeur à celui-ci, l'appareil comprenant un connecteur (6) conçu pour distribuer un gaz d'essai et une vapeur, l'appareil comportant également :
a) un ensemble de chauffage (30) pour effectuer l'évaporation d'un liquide pour produire la vapeur ;
b) un système de distribution de liquide (12, 14) conçu pour alimenter l'ensemble de chauffage en liquide ;
c) un système de distribution de gaz d'essai (18) conçu pour distribuer le gaz d'essai au connecteur ; et
d) un dispositif de commande (26) pour commander l'ensemble de chauffage (30), le système de distribution de liquide (12, 14) et le système de distribution de gaz d'essai (18) ;
**caractérisé en ce que** le connecteur comprend une chambre de mélange (30) destinée à recevoir le liquide et le gaz d'essai, et une buse (8) conçue et configurée pour se loger dans une ouverture de réception dans l'analyseur de gaz ;
et **en ce que** le connecteur (6) comporte ledit ensemble de chauffage, de telle sorte que le mélange vapeur/gaz d'essai sortant de la buse (8) soit chauffé dans le connecteur et distribué à l'analyseur de gaz sans traverser aucun autre conduit.

2. Appareil selon la revendication 1, dans lequel ledit ensemble de chauffage (30) comporte un dispositif de chauffage électrique.

3. Appareil selon l'une quelconque des revendications précédentes, dans lequel ledit ensemble de chauffage (30) entoure sensiblement la chambre (30).

4. Appareil selon l'une quelconque des revendications précédentes, dans lequel ledit gaz comprend un gaz d'essai.

5. Appareil selon l'une quelconque des revendications précédentes, dans lequel ledit système de distribution de liquide comprend une pompe volumétrique (12).

6. Appareil selon la revendication 5, dans lequel ladite pompe volumétrique (12) comprend une seringue à déplacement positif.

7. Appareil selon la revendication 7, dans lequel ladite pompe volumétrique (12) est actionnée par un moyen pour provoquer une distribution graduelle du liquide, et de préférence dans lequel ledit moyen pour provoquer une distribution graduelle dudit liquide comprend un moteur pas à pas.

8. Appareil selon l'une quelconque des revendications précédentes, dans lequel ledit système de distribution de liquide comprend en outre un conduit (32) conçu pour se raccorder audit connecteur (6).

9. Appareil selon la revendication 8, dans lequel ledit conduit comprend un tube capillaire (32).

10. Appareil selon l'une quelconque des revendications précédentes, dans lequel ledit système de distribution de gaz d'essai comprend un deuxième conduit (34) conçu pour alimenter ledit analyseur de gaz en gaz d'essai et/ou en air, et de préférence dans lequel ledit gaz d'essai et/ou air est régulé au moyen d'au moins un régulateur de débit massique.

11. Système selon les revendications 8 et 10, dans lequel ledit conduit (34) conçu pour transporter ledit liquide et le deuxième conduit sont retenus dans un tube flexible.
